# EUROPEAN PATENT SPECIFICATION

(11) **EP 1 201 252 B1**
(45) Date of publication and mention of the grant of the patent: **07.04.2004**
(21) Application number: 00122386.6
(22) Date of filing: 25.10.2000
(51) Int. Cl.: A61L 2/02, A23L 3/015, A23L 3/10

(54) **Method for inactivating microorganisms using high pressure processing**
Methode zur Inaktivierung von Mikroorganismen mittels Hochdruckverfahren
Procédé d'inactivation des micro-organismes utilisant un traitment sous haute pression

(43) Date of publication of application: 02.05.2002
(73) Proprietor: März, Andreas, 83024 Rosenheim (DE)
(72) Inventor: März, Andreas, 83024 Rosenheim (DE)
(74) Representative: VOSSIUS & PARTNER

(56) References cited:
- WO-A-97/21361
- WO-A-99/29187
- FR-A- 2 779 035
- US-A- 5 213 029
- US-A- 5 585 076
- SALE A ET AL: "Inactivation of Bacterial spores by hydrostatic pressure" JOURNAL OF GENERAL MICROBIOLOGY,GB,SOCIETY FOR MICROBIOLOGY, READING, vol. 60, 1970, pages 323-334,334A, XP002090371 ISSN: 0022-1287

## Description

This invention is related to methods for inactivating microorganisms using high pressure processing. The methods of the present invention may be applied preferably to food, cosmetic or pharmaceutical products.

Presently, the most conventional and widely used technique for inactivating microorganisms and for sterilising products is thermal processing. Thermal sterilisation methods, such as the autoclaving of canned foods, are commonly used to extend the shelf life of food products. In the medical field, thermal processing is employed in sterilising, for example, various medical instruments. Although conventional thermal sterilisation methods are quite effective for inactivating a wide range of both vegetative and non-vegetative forms of microorganisms on many types of products, there are several disadvantages. For example, thermal sterilisation often affects the natural taste, colour and/or texture of many products. Further, there are products which cannot be sterilised using conventional thermal processing because the structure would be destroyed. Thus, there is a need for effective alternative methods for inactivating microorganisms that involve limited heat treatment and moreover do not affect many of the desired substances in products.

As an alternative to conventional thermal sterilisation techniques, the effectiveness of high pressure processing at ambient temperatures for inactivating microorganisms in food has been known since the early 1900s. Although the inactivation of microorganisms at high pressures is not completely understood, it is generally believed that microorganisms are destroyed through altered permeability of the cell membranes from mechanical disruptions or through protein denaturation due to the disruption of hydrophobic and ionic bonds, and subsequent unfolding of the protein source. High pressure processing has demonstrated several advantages over conventional thermal sterilisation methods. Heat treatment of the product can be avoided or limited. The natural taste, colour and texture of many products are preserved, as well as the nutritional value and vitamin content. Further, chlorophyll and most aroma substances remain intact after high pressure processing. Physical damage to the food is also less using high pressure processing,

Japan introduced the first commercial food products to be sterilised using high pressure processing in 1990. Since then the high pressure sterilisation of foods has become widely accepted, and presently, there are several high pressure processed products on the market, including fruit, yoghurt, jam, jellies and fruit sauces.

Commercialised high pressure processing methods in the food industry generally involve placing food packed in a container inside a pressure vessel which contains a pressure transmitting medium (e.g. water). After the vessel is closed, the pressure is raised to a desired level by pumping the transmitting medium into the vessel by means of an external pressure intensifier. The vessel temperature can be maintained usually between +10 and +70 °C. The pressure is kept for a required time period. Then, the pressure is decreased. High pressure processing methods tend to vary depending on the settings for the pressure, temperature, time period and transmitting medium. Since the temperature of the vessel may be set, high pressure processing may also involve a combined application of elevated temperatures and high pressures.

In general, it has been found that pressure levels of approx. 600 MPa at 25°C are sufficient to totally inactivate vegetative forms of microorganisms. Low pressure (400-600 MPa) applied for a restricted time period (5-10 min at 25°C) has proved effective in the destruction of common contaminating microorganisms in food. In the case of pathogenic vegetative forms of microorganisms, total inactivation required slightly higher levels of processing (500-600 MPa). To destroy yeast and mould, lower levels of pressure/time period were sufficient at 25°C. Thus, high pressure processing at ambient temperatures has been proven to be very effective for inactivating non-spore forming pathogens, vegetative bacteria, yeast and moulds.

However, the effects of high pressures at ambient temperatures on the destruction of non-vegetative forms of microorganisms, e.g. bacterial spores, has proven to be limited. Some research in the field indicates that the inactivation of such microorganisms is possible using a combination of high pressures and elevated temperatures. For example, the inactivation of clostridia spores, bacilli and heat resistant moulds in technologically short time periods proved to be possible with processing at 900 MPa and simultaneously conditioning between 50 and 80 °C.

U.S. Patent No. 6,086,936 describes a method for sterilising foods using both ultra-high pressures and high temperatures. The method involves heating a food to a pre-pressurised temperature, subjecting the food to ultra-high pressure, which instantaneously raises the temperature of the food, and then releasing the pressure so that the temperature returns to the original pre-pressurised temperature. The method leverages the adiabatic temperature rise which occurs when the food is hydrostatically pressurised, coupled with the lethality of the pressure, to achieve appropriate sterilisation conditions. The method disclosed however seems to be restricted to food, in particular non-dairy food products having a pH equal to or greater than 4.6.

In the cosmetic and pharmaceutical industries, there is a need for effective sterilisation techniques which do not reduce or alter the potency of the active substances in the product. Due to the high temperatures employed, it is not possible to use conventional heat sterilisation methods on many temperature-sensitive pharmaceutical products without negatively affecting the efficacy of the active substance. Therefore, a more gentle and equally effective means for sterilising such products would represent a significant technical advantage in the field. The potential to process cosmetic and pharmaceutical products using high pressure processing is already recognised as a viable option to current methods. However, an optimal method for inactivating microorganisms in cosmetic and pharmaceutical products has not been disclosed by the prior art.

In "Pressure inactivation of microorganisms at moderate temperatures" (Butz, P and Ludwig, H; Physica 139&140 B, 1986), "High Pressure inactivation of bacteria and bacteria spores" (Butz, P, Ludwig, H et al; Pharm. Ind. 52, 1990), "Pressure induced germination and inactivation of Bacillus subtillis spores" (Sojka, B and Ludwig, H; Pharm. Ind. 56, 1994) and "Pressure and temperature induced inactivation of microorganisms" (Ludwig, H, Scigalla,W and Sojka, B; High Pressure Effects in Molecular Biophysics and Enzymology-Northrop and Royer, Chapter 22, 1996), methods using a combination of high pressures and elevated temperatures are discussed for inactivating microorganisms, primarily bacteria and bacterial spores, with applications to the drug industry. Various parameters are investigated in order to ascertain the optimal conditions for destroying the microorganisms mentioned. For example, in the article from Sojka and Ludwig in Pharm. Ind of 1994, the pressure inactivation of bacterial spores was studied in the range from 600 to 6000 bar at temperatures of 40 and 50 °C. At 40°C, the number of germs could be reduced by a factor of 10⁶ after 210 min. of pretreatment at 600 bar and following inactivation at 5000 bar for some minutes. Raising the temperature to 50°C, an alternating pressurisation at 600 and 5000 bar in intervals of 30 min. led to the complete inactivation of spores after an overall time of 180 min. In the aforementioned "Pressure and temperature induced inactivation of microorganisms" of 1996, results showed that pressure cycles are useful for the inactivating spores and further that the addition of ethanol promotes inactivation.

In the aforementioned references, several options are considered for yielding a method for destroying specific bacteria and bacterial spores whilst at the same time minimising the parameters of temperature, pressure and duration of the pressure applied. However, optimal parameters for a method of inactivating a wide range of microorganisms, including non-vegetative forms, using high pressure processing that may also be safely applied to pharmaceutical products is yet to be determined.

It is therefore an object of the present invention to provide a more effective method for inactivating microorganisms using high pressure processing.

The object of the present invention is achieved with the features of the claims.

The subject-matter according to the present invention has the particular advantage that it can be safely applied to pharmaceutical and cosmetic products without affecting the efficacy of the active substances in the products.

Another advantage of the present invention exists in providing an innovative method for inactivating microorganisms using high pressure processing that is effective for inactivating even difficult-to-destroy non-vegetative bacteria and bacterial spores and at the same time, requires lower temperatures, lower pressures and shorter pressure application time periods as that of known methods.

The present invention provides an improved method for inactivating microorganisms using high pressure processing. A combination of high pressures and elevated temperatures are used to inactivate even worst case bacterial spores. Although heat is applied to the product, the heat applied is generally significantly lower than that of conventional thermal sterilisation processes. Thus, the present method would still be considered as more desirable than conventional thermal processes for heat-sensitive products.

Further, the methods of the present invention introduce new factors which are used to minimise the high pressure processing parameters necessary for inactivating specific microorganisms, such as the required temperature and pressure applied to the product. It has been found that the effects of high pressure processing on the inactivation of microorganisms may be greatly enhanced by pre-treating the product with specified amounts of oxygen. Since oxygen pre-treatment can promote inactivation, the processing parameters required in order to achieve the same microorganism reduction criteria can be somewhat lower than the parameters required in methods without pre-treatment. In other words, lower processing temperatures and pressures at shorter time periods can be employed.

It was recognised surprisingly by experimentation that, when using products packed in flexible containers, oxygen pathways resulting from the high pressures of the transmitting medium applied appear to influence the amount of microorganisms reduced. The presence of oxygen pathways contributed to increasing the overall effectiveness of the inactivation process. Thus, it is believed that by pre-treating the product using specified amounts of oxygen, one can further increase the effectiveness of the high pressure processing methods thereby simultaneously allowing a reduction of the processing parameters.

It is well-known that oxygen is highly toxic because oxidation reactions can inactivate enzymes. An improved method which incorporates this factor can provide a very effective way to inactivate microorganisms requiring minimised processing parameters, i.e. lower temperatures and pressures at shorter time intervals as that of known methods. Methods requiring minimised processing parameters are more desirable when processing sensitive products such as pharmaceuticals.

Another surprising factor is the influence of the various types of packaging on the high pressure processing methods. Several containers were tested and not all conventional containers were proven to be suitable under particular processing conditions. Thus, a consideration of the appropriate packaging has to be made in order to assure minimal damage to the product.

According to the present invention is a method for inactivating microorganisms using high pressure processing comprising the steps of; (i) subjecting the product to a predetermined amount of oxygen for a time interval; (ii) heating a product to a pre-pressurised temperature; (iii) placing the product in a pressure vessel; (iv) subjecting the product to a pressure at a pressurised temperature for a time period in the presence of a transmitting pressure fluid; (v) reducing the pressure in the vessel after said time period; (vi) removing the product from said pressure vessel, wherein said method steps (iv) and (v) may be repeated at least once before step (vi) is performed, said time period and said pressure being adjustable between repetitions of steps (iv) and (v).

The product is heated to a pre-pressurised temperature using preferably a water bath. The pre-pressured temperature is preferably greater than ambient temperature and less than 70 °C. The transmitting pressure fluid in the pressure vessel is preferably water.

The pressure in the pressure vessel is preferably between 500 and 8000 bar, and preferably able to be incrementally increased and decreased. The pressurised temperature is preferably between 20 and 100 °C.

During the high pressure process, the pressure has a uniform effect on the entire pre-packaged product. The flexibility of the product's container enables it to compensate for external pressure through a reduction in volume. Thus, the containers used in the present method are flexible.

The transmitting pressure fluid in the pressure vessel is preferably water.

The present invention can be utilised to process a variety of products, especially food, cosmetic and pharmaceutical products.

Using the following figures, preferred embodiments of the invention are described in further detail:
Figure 1: shows a conventional high pressure processing device
Figure 2: illustrates a pressure vessel used in a conventional high pressure processing device

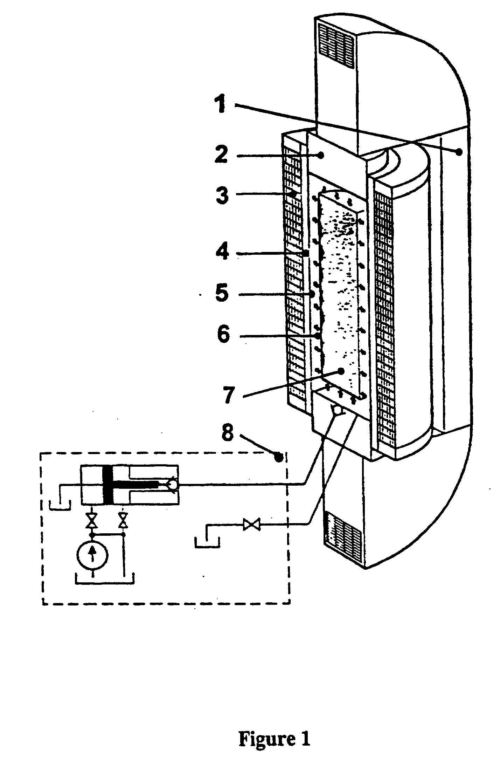

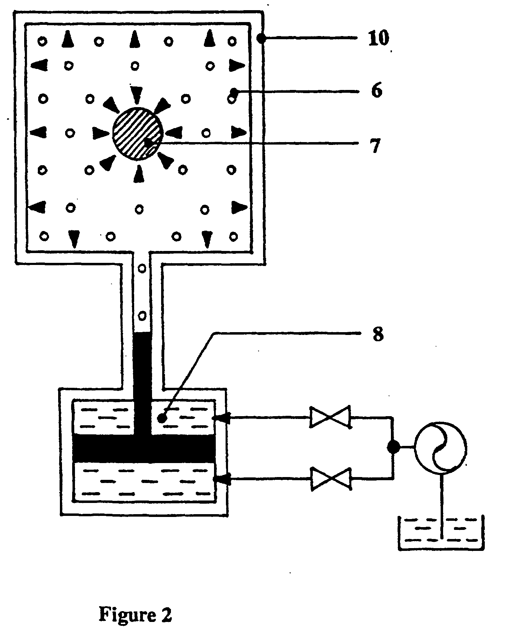

According to the present invention, a pre-packaged and pre-exposed to oxygen product 7 is initially heated in preferably a water bath until a pre-pressurised temperature is reached. This temperature is preferably between 30 and 70 °C. Referring to Figures 1 and 2, the pre-packaged product 7 is then placed into a pressure vessel 10 located within a pressure cylinder 4. The pressure vessel 10 contains a pressure transmitting medium 6, preferably water. After the pressure vessel 10 is closed, the pressure is raised to a desired level by pumping the transmitting medium 6 into the pressure vessel 10 by means of an external pressure intensifier 8. Once the desired pressures and temperatures are achieved in the pressure vessel 10, they are maintained for a predetermined time period. After the time period has expired, the pressure and temperature are reduced. The pre-packaged product is then removed from the pressure vessel 10.

The pre-packed product can also be processed using intervals having different or the same processing parameters. For example, after the first high pressure process (i.e. previously mentioned steps c) and d)), the product can be processed again before it is removed from the pressure vessel 10 by resetting the parameters. To allow this, the pressure in the pressure vessel 10 may be increased or decreased. Once the newly set pressure is achieved in the pressure vessel 10, it is maintained for the newly set time period.

In order to inactivate a wide range of microorganisms using the methods of the present invention, several processing parameters were experimentally tested in order to determine effective and robust methods.

### Experimental Results

For the protection of the consumer, pharmaceutical preparations must meet several microbiological purity criteria. The criteria for the various pharmaceutical products differ depending on the category to which they belong. Products that have a greater potential for harming the consumer are placed in categories having stricter criteria.

In order to meet such criteria, a wide range of microorganisms were experimentally tested ranging from vegetative bacteria to bacterial spores. The tested bacteria included Staphlococcus aureus (STA), Escherichia coli (EC), Pseudomonas aeruginosa (PSA), Candida albicans (CA), Clostridium sporogenes (CL), Bacillus subtillus (BS) and Aspergillus niger (AN).

Firstly, concentrations of the bacteria were made by culturing according to standard techniques. The samples were prepared by mixing the bacteria with a salt solution in order to obtain a concentration of 10⁶ KBE/ml. The following 5 samples were tested:
A: a mixed suspension of EC and PSA
B: a mixed suspension of STA and BS
C: a mixed suspension of CA and AN
D: CL(1)
E: CL(2)

Two samples of clostridium sporogenes were prepared, so that the processing parameters necessary for inactivating these spores could be tested for robustness. These spores were considered as difficult-to-destroy and thus termed the worst case bacteria.

Each of the samples were filled into five different types of containers, so that the feasibility of using the various conventional containers could also be tested. Each sample was filled into 1) a 20 ml PE screwable bottle, 2) a 30 ml PE tube, 3) a 10ml PE-AT vial, 4) a 20 ml PE-ampoule and 5) a 20 ml flexible film container.

The following tests are not according to the present invention because the samples were not subjected to a predetermined amount of oxygen prior to the heating step. They are, however, representative of the steps following said oxygen pretreatment.

The test results of two different processing parameters are shown in Tables 1, and 2. Tables 1 and 2 illustrate the reduction factors in log₁₀ for each of the tested microorganisms after the samples were processed.

Referring to Table 1, the pre-packaged sample was first heated in a water bath until a pre-pressured temperature of 32°C was achieved. The sample was then placed in a pressure vessel 10. The pressure in the pressure vessel 10 was brought to 6000 bar, and the sample was pressurised at a temperature of 60°C. After a time period of 300 min, the pressure was reduced.

A reduction factor greater than 10⁶ (>6) was considered adequate for reaching acceptable microorganism inactivation conditions. As seen in Table 1, these parameters were not adequate for inactivating clostridium sporegenes in all containers tested and bacillus subtillus in the flexible film containers. However, bacillus subtillus was suitably reduced in other containers. Further, the other tested bacteria was suitably reduced in all containers.

Referring to Table 2, the pre-packaged sample was first heated in a water bath until a pre-pressured temperature of 50°C was achieved. The sample was then placed in a pressure vessel 10. The pressure in the pressure vessel 10 was brought to 6000 bar and the sample was pressurised at a temperature of 60°C. After a time period of 180 min, the pressure was reduced.

**Table 1**

| Samples | Reduction factors in log₁₀: parameters (300 min, 6000 bar, 60°C) | | | | | | | |
|---|---|---|---|---|---|---|---|---|
| | RF_{EC60} | RF_{PSA60} | RF_{STA60} | RF_{BS60} | RF_{CA60} | RF_{AN60} | RF_{CL60(1)} | RF_{CL60(2)} |
| 20 ml-PE-Screwable Bottle | > 6 | > 6 | > 6 | > 6 | > 6 | > 6 | 0 | 1 |
| 30 ml-PE-Tube | > 6 | > 6 | > 6 | > 6 | > 6 | > 6 | 1 | 1 |
| 10 ml-PE-AT-Vial | > 6 | > 6 | > 6 | > 6 | > 6 | > 6 | 1 | 1-2 |
| 20 ml-PE- Ampoule | > 6 | > 6 | > 6 | > 6 | > 6 | > 6 | 1-2 | 2 |
| 20 ml-film container | > 6 | > 6 | > 6 | 5 | > 6 | > 6 | 1 | 1-2 |

**Table 2**

| Samples | Reduction factors in log₁₀: parameters (180 min, 6000 bar, 90°C) | | | | | | | |
|---|---|---|---|---|---|---|---|---|
| | RF_{EC90} | RF_{PSA90} | RF_{STA90} | RF_{BS90} | RF_{CA90} | RF_{AN90} | RF_{CL90(1)} | RF_{CL90(2)} |
| 20 ml-PE-Screwable Bottle | > 6 | > 6 | > 6 | > 6 | >6 | >6 | >6 | >6 |
| 30 ml-PE-Tube | >6 | >6 | >6 | >6 | >6 | >6 | >6 | > 6 |
| 10 ml-PE-AT-Vial | >6 | >6 | >6 | >6 | >6 | >6 | >6 | >6 |
| 20 ml-PE- Ampoule | >6 | >6 | >6 | >6 | >6 | >6 | >6 | >6 |
| 20 ml-film container | >6 | >6 | >6 | 5 | >6 | >6 | 4 | 4 |

As seen in Table 2, these parameters prove to be effective for inactivating all the microoganisms tested. However, clostridium sporegenes in the flexible film containers were still not sufficiently inactivated. Experimental results indicate that the use of certain laminated flexible film containers under such processing conditions may hinder the effectiveness of the method and thus the containers do not play an insignificant role in the inactivation of microorganisms using high pressure processing.

## Claims

1. A method for inactivating microorganisms in a product using high pressure processing comprising the steps of:
(a) packing said product in a flexible container, wherein steps a) and b) can be in any order;
(b) heating said product to a pre-pressurised temperature;
(c) subjecting said product to a pressure at a pressurised temperature for a time period;
(d) reducing the pressure after said time period.
**characterized in that** said product is subjected before step (b) to a predetermined amount of oxygen for a time interval.

2. The method of claim 1, wherein said product is heated to the pre-pressurised temperature in a water bath.

3. The method of any of the preceding claims, wherein the pre-pressured temperature is preferably greater than 25°C and less than 70°C.

4. The method of any of the preceding claims, wherein method steps (c) and (d) are repeated at least once and said time period and said pressure being adjustable between repetitions of steps (c) and (d).

5. The method of any of the preceding claims, wherein between method steps b) and c) said product is placed in a pressure vessel and removed therefrom after step (d).

6. The method of any of the preceding claims, wherein pressure is transmitted using a pressure fluid, preferably water.

7. The method of any of the preceding claims, wherein the pressure is between 500 and 8000 bar.

8. The method of any of the preceding claims, wherein the pressure is incrementally increased or decreased.

9. The method of any of the preceding claims, wherein the pressurised temperature is between 20 and 100 °C.

10. The method of any of the preceding claims, wherein the flexible container is a polyethylene ampoule.

11. The method of any of the preceding claims, wherein said product is a pharmaceutical product.

12. The method of any of claims 1 to 10, wherein said product is a cosmetic product.

13. The method of any of claims 1 to 10, wherein said product is a food product.

## Patentansprüche

1. Verfahren zur Inaktivierung von Mikroorganismen in einem Produkt unter Verwendung von Hochdruckbearbeitung mit den folgenden Schritten:
(a) Verpacken des Produkts in einem flexiblen Behälter, wobei die Schritte (a) und (b) jede Reihenfolge haben können;
(b) Erwärmen des Produkts auf eine Vordrucktemperatur;
(c) Einwirkenlassen eines Drucks bei einer Drucktemperatur für eine Zeitperiode auf das Produkt;
(d) Reduzieren des Drucks nach der Zeitperiode,
**dadurch gekennzeichnet, daß** das Produkt vor dem Schritt (b) einer vorbestimmten Menge von Sauerstoff für ein Zeitintervall ausgesetzt wird.

2. Verfahren nach Anspruch 1, wobei das Produkt in einem Wasserbad auf die Vordrucktemperatur erwärmt wird.

3. Verfahren nach einem der vorstehenden Ansprüche, wobei die Vordrucktemperatur vorzugsweise höher als 25 °C und niedriger als 70 °C ist.

4. Verfahren nach einem der vorstehenden Ansprüche, wobei die Verfahrensschritte (c) und (d) mindestens einmal wiederholt werden und die Zeitperiode sowie der Druck zwischen Wiederholungen der Schritte (c) und (d) einstellbar sind.

5. Verfahren nach einem der vorstehenden Ansprüche, wobei zwischen den Verfahrensschritten (b) und (c) das Produkt in einem Druckgefäß plaziert ist und aus diesem nach dem Schritt (d) entnommen wird.

6. Verfahren nach einem der vorstehenden Ansprüche, wobei Druck unter Verwendung eines Druckfluids, vorzugsweise Wasser, übertragen wird.

7. Verfahren nach einem der vorstehenden Ansprüche, wobei der Druck zwischen 500 und 8000 Bar liegt.

8. Verfahren nach einem der vorstehenden Ansprüche, wobei der Druck schrittweise erhöht oder verringert wird.

9. Verfahren nach einem der vorstehenden Ansprüche, wobei die Drucktemperatur zwischen 20 und 100 °C liegt.

10. Verfahren nach einem der vorstehenden Ansprüche, wobei der flexible Behälter eine Polyethylenampulle ist.

11. Verfahren nach einem der vorstehenden Ansprüche, wobei das Produkt ein pharmazeutisches Produkt ist.

12. Verfahren nach einem der Ansprüche 1 bis 10, wobei das Produkt ein kosmetisches Produkt ist.

13. Verfahren nach einem der Ansprüche 1 bis 10, wobei das Produkt ein Nahrungsmittelprodukt ist.

## Revendications

1. Procédé d'inactivation des micro-organismes dans un produit utilisant un traitement sous haute pression comprenant les étapes consistant à :
a) emballer ledit produit dans un récipient souple, dans lequel les étapes a) et b) peuvent être réalisées dans n'importe quel ordre ;
b) chauffer ledit produit à une température pré-pressurisée ;
c) soumettre ledit produit à une pression à une température pressurisée pendant une période de temps ;
d) réduire la pression après ladite période de temps,
**caractérisé en ce que** ledit produit est soumis avant l'étape (b) à une quantité prédéterminée d'oxygène pendant un intervalle de temps.

2. Procédé selon la revendication 1, dans lequel ledit produit est chauffé à la température pré-pressurisée dans un bain d'eau.

3. Procédé selon l'une quelconque des revendications précédentes, dans lequel la température pré-pressurisée est de préférence supérieure à 25°C et inférieure à 70°C.

4. Procédé selon l'une quelconque des revendications précédentes, dans lequel les étapes (c) et (d) du procédé sont répétées au moins une fois et ladite période de temps et ladite pression étant réglables entre les répétitions des étapes (c) et (d).

5. Procédé selon l'une quelconque des revendications précédentes, dans lequel entre les étapes b) et c) du procédé, ledit produit est placé dans un récipient de pression et retiré de celui-ci après l'étape d).

6. Procédé selon l'une quelconque des revendications précédentes, dans lequel la pression est transmise en utilisant un fluide sous pression, de préférence de l'eau.

7. Procédé selon l'une quelconque des revendications précédentes, dans lequel la pression est comprise entre 500 et 8000 bars.

8. Procédé selon l'une quelconque des revendications précédentes, dans lequel la pression est augmentée ou diminuée de manière incrémentielle.

9. Procédé selon l'une quelconque des revendications précédentes, dans lequel la température pressurisée est comprise entre 20 et 100°C.

10. Procédé selon l'une quelconque des revendications précédentes, dans lequel le récipient souple est une ampoule de polyéthylène.

11. Procédé selon l'une quelconque des revendications précédentes, dans lequel ledit produit est un produit pharmaceutique.

12. Procédé selon l'une quelconque des revendications 1 à 10, dans lequel ledit produit est un produit cosmétique.

13. Procédé selon l'une quelconque des revendications 1 à 10, dans lequel ledit produit est un produit alimentaire.
